# EUROPÄISCHE PATENTSCHRIFT

(11) **EP 0 570 817 B1**
(45) Veröffentlichungstag und Bekanntmachung des Hinweises auf die Patenterteilung: **23.04.1997**
(21) Anmeldenummer: 93107672.3
(22) Anmeldetag: 11.05.1993
(51) Int. Cl.: C07D 209/34, C07C 205/12

(54) **Verfahren zur Herstellung von 5-Chloroxindol**
Process for the preparation of 5-chloroxindole
Procédé pour la préparation du 5-chloroxindol

(30) Priorität: 13.05.1992 CH 1528/92
(43) Veröffentlichungstag der Anmeldung: 24.11.1993
(73) Patentinhaber: LONZA AG, CH-3945 Gampel/Wallis (CH)
(72) Erfinder: Imwinkelried, René, Dr., Brig-Glis, (Kanton Wallis) (CH); Previdoli, Felix, Dr., Brig (Kanton Wallis) (CH)
(74) Vertreter: Weinhold, Peter, Dr.

(56) Entgegenhaltungen:
- US-A- 3 882 236
- US-A- 4 160 032
- US-A- 4 721 712
- US-A- 4 730 004
- US-A- 4 761 485
- J.ORG.CHEM. Bd. 51, Nr. 10, 28. Januar 1986, Seiten 1704 - 1712 RAJANBABU ET AL 'alpha-Nitroarylation of Ketones and esters'
- SYNTHESIS Bd. 1, Nr. 93, 1. Januar 1993, Seiten 51 - 53 QUALLICH 'A General Oxindole Synthesis' Verbindungen 2a,3a,4a,5a

## Beschreibung

Die Erfindung betrifft ein neues Verfahren zur Herstellung von 5-Chloroxindol der Formel 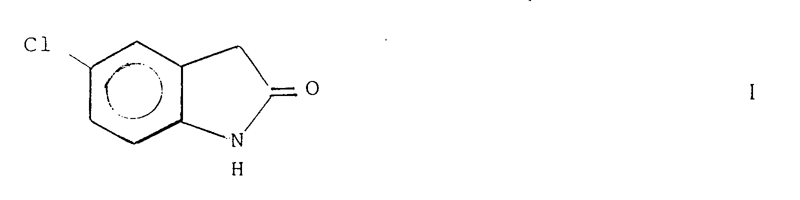 ausgehend von Chlornitrobenzol.

5-Chloroxindol ist ein wichtiges Zwischenprodukt zur Herstellung von Pharmazeutika wie beispielsweise zur Herstellung von 1,3-disubstituierten 2-Oxoindolen (US-A-4 721 712).

Bisher sind mehrere Verfahren zur Herstellung von 5-Chloroxindol bekannt.

Quallich et al. (Synthesis, 1, 51-53; 1993) beschreiben eine dreistufige Synthese von 5-Chloroxindol, bei der zunächst ein 2,4-Dichlornitrobenzol mit Dimethylmalonat zu einem Dimethyl(5-chlor-2-nitrophenyl)malonat umgesetzt wird, das anschließend einer Decarboxylierung unterworfen wird. Das erhaltene Acetat wird dann reduktiv zum gewünschten Endprodukt cyclisiert.

Die US-A-4 761 485 beschreibt, ein Verfahren zur Herstellung von 5-Chloroxindol ausgehend von 5-Chlorindol. Hierbei wird zunächst 5-Chlorindol mittels Pyridinbromidperbromid in 3,3-Dibrom-5-chloroxindol überführt, welches dann mittels katalytischer Hydrierung mit Palladium auf Kohle in 5-Chloroxindol überführt wird.

Ein Nachteil dieses Verfahrens besteht darin, dass das Edukt (5-Chlorindol ) schwer zugänglich ist und dass 5-Chloroxindol in schlechter Ausbeute erhalten wird.

Des weiteren beschreibt die US-A-4 730 004 ein Verfahren zur Herstellung von 5-Chloroxindol ausgehend von 5-Chlorisatin, wobei dieses zunächst mit Hydrazinhydrat in das 5-Chlor-3-hydrazon-2-oxoindol überführt wird, welches dann mittels Natriummethanolat in 5-Chloroxindol umgesetzt wird.

Auch dieses Verfahren hat den Nachteil, dass das Edukt 5-Chlorisatin schwer zugänglich ist und dass das gewünschte Produkt in schlechter Ausbeute erhalten wird.

Die US-A-4 160 032 beschreibt ein Verfahren zur Herstellung von 5-Chloroxindol, bei dem man eine 5-Chlor-2-nitrophenylessigsäure in Gegenwart eines Platinkatalysators einer reduktiven Zyklisierung unterwirft. Die 5-Chlor-2-nitrophenylessigsäure wird ihrerseits hergestellt, indem man 3-Chlorphenylessigsäure als Ausgangsprodukt in Gegenwart konzentrierter Schwefel- und Salpetersäure im Verlauf von 24 Stunden nitriert. Nachteilig hierbei ist nicht nur die lange Umsetzungszeit bei der Nitrierung, sondern auch, daß 3-Chlorphenylessigsäure kommerziell nicht erhältlich ist und erst in einem separaten Reaktionsschritt hergestellt werden muß. Das beschriebene Verfahren ist daher unwirtschaftlich und für ein großtechnisches Verfahren nicht geeignet.

RajanBabu et al. (J. Org. Chem., 51, 1704-1712; 1986) beschreiben ein Verfahren zur Herstellung von 2-Oxoindolen durch reduktive Zyklisierung von Halonitrophenylessigsäurealkylestern. Die Herstellung dieser Ester kann allgemein durch Umsetzung eines Halonitrobenzols mit einem von einem Ester oder Keton abgeleiteten Silylenolether und durch anschließende Oxidation des erhaltenen Reaktionsprodukts erfolgen. Die Silylenolether sind jedoch äußerst wasserempfindlich und in größeren Mengen schwer erhältlich. Aus diesem Grund ist auch dieses Verfahren für den großtechnischen Maßstab nicht geeignet.

Die Aufgabe der Erfindung war, ein wirtschaftliches und einfaches Verfahren zur Herstellung von 5-Chloroxindol zur Verfügung zu stellen, wobei das Produkt in guter Ausbeute erhalten wird.

Diese Aufgabe wird erfindungsgemäss mit dem neuen Verfahren gelöst.

Erfindungsgemäss wird das Verfahren zur Herstellung von 5-Chloroxindol der Formel 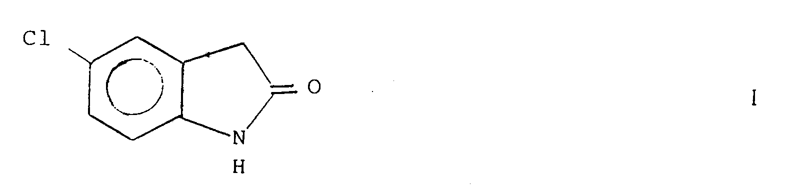 derart durchgeführt, dass man in der ersten Stufe p-Chlornitrobenzol der Formel 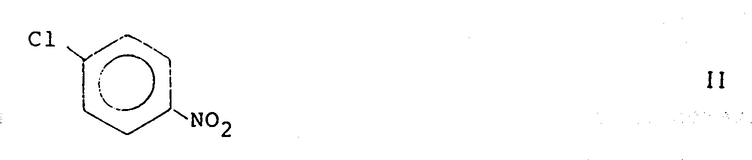 mit einem Chloressigsäurealkylester der allgemeinen Formel 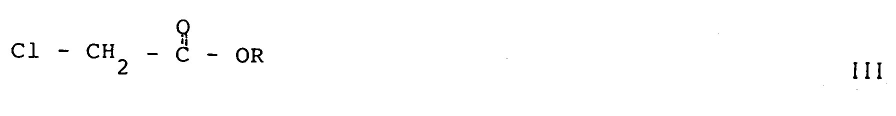 worin R eine C₁-C₇-Alkylgruppe, verzweigt oder unverzweigt, bedeutet, in Gegenwart einer Base zu einem Chlornitrobenzolessigsäurealkylester der allgemeinen Formel 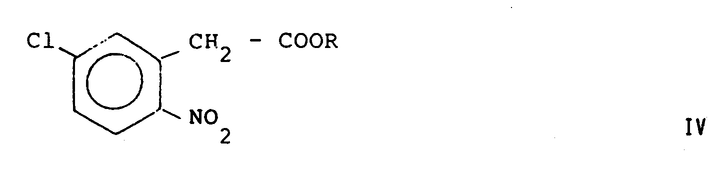 worin R die genannte Bedeutung hat, überführt, diesen in der zweiten Stufe katalytisch mit Wasserstoff zu dem entsprechenden Amin der allgemeinen Formel 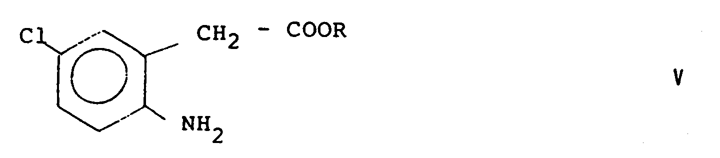 worin R die genannte Bedeutung hat, hydriert und dieses dann in der dritten Stufe in Gegenwart einer Säure zum Endprodukt gemäss Formel I cyclisiert.

Die erste Stufe wird mit p-Chlornitrobenzol der Formel II und mit einem Chloressigsäure-C₁-C₇-alkylester der allgemeinen Formel III durchgeführt.

Als zweckmässige Vertreter der Chloressigsäure-C₁-C₇-alkylester werden diejenigen angewendet, worin C₁-C₇ eine Methyl-, Ethyl-, Propyl-, Isopropyl-, tertiär Butyl- oder tertiär Pentylgruppe bedeutet.

Vorzugsweise bedeutet C₁-C₇ eine Ethylgruppe.

Vorzugsweise wird der Chloressigsäure-C₁-C₇-alkylester im Überschuss bezogen auf p-Chlornitrobenzol eingesetzt, vorzugsweise in einer Menge von 1,3 bis 1,7 mol bezogen auf 1 mol Chlornitrobenzol.

Als Lösungsmittel dieser beiden Reaktanten können unpolare Lösungsmittel wie beispielsweise Toluol, Diethylether, Tetrahydrofuran oder tertiär Butylmethylether angewendet werden. Vorzugsweise wird Toluol angewendet.

Die erste Stufe wird in Gegenwart einer Base durchgeführt. Als Base können beispielsweise Alkaliamide oder Alkalihydroxide verwendet werden.

Als Alkalihydroxid wird beispielsweise Natrium- oder Kaliumhydroxid angewendet.

Als Alkaliamid wird beispielsweise Natrium- oder Kaliumamid verwendet.

Zweckmässig wird als Base ein Alkaliamid in flüssigem Ammoniak, vorzugsweise Natriumamid, welches insbesondere in situ aus dem entsprechenden elementaren Metall in flüssigem Ammoniak, gegebenenfalls in Gegenwart eines Katalysators, gebildet wird.

In einer besonders bevorzugten Ausführungsform der ersten Stufe wird das in situ gebildete Alkaliamid in Gegenwart eines Alkohols der allgemeinen Formel

R - OH VI

worin R die genannte Bedeutung hat, eingesetzt. Als zweckmässige Vertreter dieser Alkohole werden die angewendet, worin R Methyl-, Ethyl-, Propyl-, Isopropyl, tertiär Butyl oder tertiär Pentyl bedeutet, vorzugsweise tertiär Butyl.

Vorzugsweise wird der Alkohol und das Alkaliamid äquimolar eingesetzt.

Zweckmässig wird die Umsetzung in der ersten Stufe bei einer Temperatur von -30 bis -40°C durchgeführt.

Nach einer üblichen Umsetzungsdauer von 0,1 bis 2 h kann durch Zugabe von Ammoniumchlorid der Chlornitrobenzolessigsäurealkylester gemäss Formel 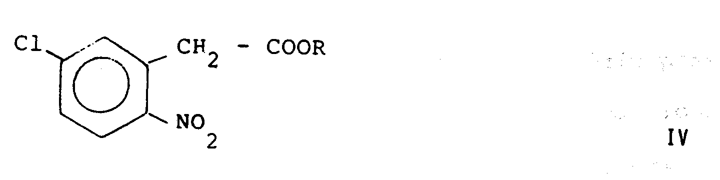 auf fachmännisch übliche Weise isoliert werden.

Die zweite Stufe, die Hydrierung des Chlornitrobenzolessigsäurealkylesters (Formel IV) zu dem entsprechenden Amin (Formel V) wird katalytisch mit Wasserstoff durchgeführt.

Als Hydrierkatalysatoren können Edelmetall-, Edelmetalloxid oder Raney-Katalysatoren, gegebenenfalls aufgebracht auf einem geeigneten Trägermaterial angewendet werden.

Beispielsweise können als Hydrierkatalysatoren Raney-Nickel oder Platin auf Kohle angewendet werden.

Zweckmässig wird als Hydrierkatalysator Platin auf Kohle, insbesondere 0,5 bis 5 Gew.% Platin auf Kohle, angewendet.

Die Hydrierkatalysatoren können in einer Menge von 0,1 bis 20 Gew.%, vorzugsweise von 5 bis 10 Gew.%, bezogen auf Chlornitrobenzolessigsäurealkylester angewendet werden.

Zweckmässig erfolgt die Hydrierung bei einem erhöhten H₂-Druck, vorzugsweise bei einem Druck von 5 bis 10 bar.

Zweckmässig wird die zweite Stufe in apolarem Lösungsmittel, wie z.B. Toluol, oder in einem polaren Lösungsmittel, wie beispielsweise in Alkoholen oder in Estern, durchgeführt.

Als Ester können beispielsweise Essigsäuremethyl- oder Essigsäureethylester verwendet werden.

Als Alkohole können beispielsweise Methanol, Ethanol oder Propanol angewendet werden. Vorzugsweise wird Ethanol angewendet.

Zweckmässig wird die Umsetzung in der zweiten Stufe bei einer Temperatur von 0 bis 55°C, vorzugsweise von 10 bis 20°C, durchgeführt.

Nach einer üblichen Umsetzungszeit von 1 bis 20 h kann dann das Amin (Formel V) entweder nach fachmännisch üblichen Methoden isoliert oder nach Abtrennen des Katalysators direkt für die dritte Stufe eingesetzt werden. Vorzugsweise wird das Amin, ohne Isolation, direkt für die dritte Stufe eingesetzt.

In der dritten Stufe wird das Amin der allgemeinen Formel V in Gegenwart einer Säure zu 5-Chloroxindol (Formel I) cyclisiert.

Als Säuren können beispielsweise Toluol-4-sulfonsäure, Methansulfonsäure oder deren Hydrate angewendet werden. Vorzugsweise wird Toluol-4-sulfonsäure oder deren Hydrate verwendet.

Zweckmässig wird die Säure in einer Menge von 0,0005 bis 0,1 mol, vorzugsweise von 0,05 bis 0,1 mol, pro mol Amin eingesetzt.

Als Lösungsmittel können für die dritte Stufe die gleichen wie die, die für die zweite Stufe beschrieben sind, eingesetzt werden.

Die Umsetzung in der dritten Stufe erfolgt zweckmässig bei einer Temperatur von 50°C bis Rückflusstemperatur vorzugsweise von 70°C bis zur Rückflusstemperatur des Lösungsmittels.

Nach einer üblichen Umsetzungszeit von 1 bis 20 h kann 5-Chloroxindol nach fachmännisch üblichen Methoden in guter Ausbeute isoliert werden.

### Beispiel

### Herstellung von 5-Chloroxindol

### (a) Herstellung von Chlornitrobenzolessigsäureethylester (Erste Stufe)

- 250 ml: NH₃ wurden in einen getrockneten und mit Argon gespülten Kolben (Kryomattemperatur -40°C) einkondensiert. Ein kleines Stückchen Natrium wurde zugegeben -> blaue Lösung. Nach Zugabe von
- 250 mg: Eisen(III)nitrat-Nonahydrat entfärbte sich die Lösung.
Innerhalb 15 Minuten wurden
- 5.75 g: (250 mmol) Natrium in kleinen Stücken zugegeben. Anschliessend wurde 10 Minuten gerührt. Dann wurden
- 18.53 g: (250 mmol) tert. Butanol, gelöst in
- 3 ml: Toluol, während 15 Minuten zugetropft und 35 Minuten gerührt -> graue Suspension.
Gleich danach wurde eine Mischung von
- 15.76 g: (100 mmol) 4-Chlornitrobenzol und
- 18.38 g: (150 mmol) Chloressigsäureethylester, gelöst in
- 20 ml: Toluol, während 15 Minuten zugetropft (Reaktionsgemisch wurde blau). Nach der Zugabe wurde eine Stunde weitergerührt, dann wurden
- 26.75 g: (500 mmol) festes Ammoniumchlorid vorsichtig zugegeben. Anschliessend wurde der Trockeneiskühler entfernt und die Suspension innerhalb ca. 30 Minuten auf 10°C erwärmt (Entfernung von NH₃). Innerhalb 20 Minuten wurden dann
- 200 ml: Toluol zugetropft (Kryomattemperatur: 10°C). Nach 30 Minuten wurde das Reaktionsgemisch über eine G3-Glasfilternutsche (mit Celite) filtriert. Das Filtrat wurde am Rotavap bei 35°C und bei 25 mbar eingeengt und am Hochvakuum ca. 30 Minuten getrocknet.

Es wurden 27.14 g Produkt, Gehalt (HPLC): 78.8%, entsprechend einer Ausbeute von 87.8% bezogen auf eingesetztes Edukt erhalten.

### (b) Herstellung von Chloraminobenzolessigsäureethylester

- 26.73: g Rohprodukt aus Stufe 1 wurden in
- 135 ml: Ethanol bei Raumtemperatur gelöst. Nach Zugabe von
- 1.0 g: Pt/C wurde der Autoklav dreimal mit H₂ gespült und dann wurden 5 bar H₂ aufgepresst und während 7,5 Stunden bei Raumtemperatur gerührt. Anschliessend wurde das Reaktionsgemisch abfiltriert und der Filterrückstand mit
- 25 ml: Ethanol gewaschen.
Die so erhaltene Filterlösung wurde direkt weiterverarbeitet (dritte Stufe).

### (c) Herstellung von 5-Chloroxindol

Die Filterlösung der zweiten Stufe wurde mit
- 1.90 g: (10 mmol) Toluol-4-sulfonsäure-monohydrat versetzt. Anschliessend wurde 30 Minuten unter Rückfluss erhitzt. Während 4,5 Stunden wurden dann total 125 ml Ethanol abdestilliert, worauf eine Suspension entstand. Das Reaktionsgemisch wurde auf Raumtemperatur abgekühlt und filtriert. Der Filterrückstand wurde mit insgesamt
- 60 ml: Ethanol (3x20 ml-Portionen) gewaschen und anschliessend während 12 Stunden bei 35°C und bei 40 mbar getrocknet.

Es wurden 12.07 g rosa-violetter Feststoff als Produkt, Gehalt (HPLC): 94.3%, entsprechend einer Ausbeute von 67.9% bezogen auf eingesetztes Chlornitrobenzol (erste Stufe) erhalten.

## Patentansprüche

1. Verfahren zur Herstellung von 5-Chloroxindol der Formel 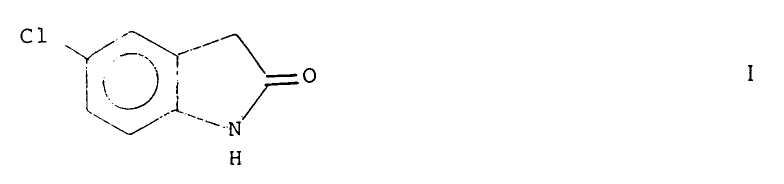 dadurch gekennzeichnet, dass man in der ersten Stufe p-Chlornitrobenzol der Formel 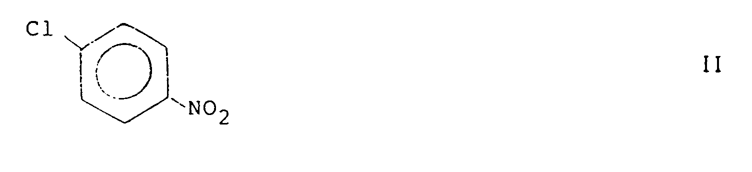 mit einem Chloressigsäurealkylester der allgemeinen Formel 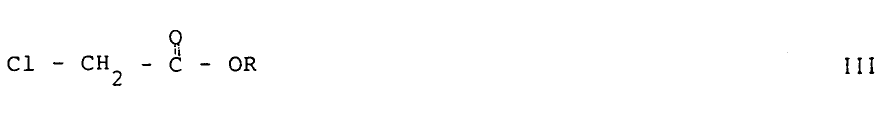 worin R eine C₁-C₇-Alkylgruppe, verzweigt oder unverzweigt, bedeutet, in Gegenwart einer Base zu einem Chlornitrobenzolessigsäurealkylester der allgemeinen Formel 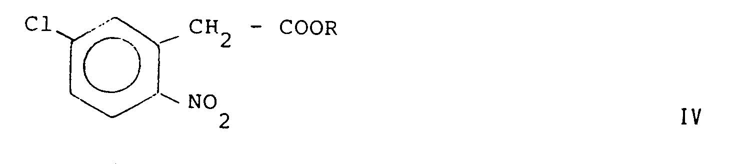 worin R die genannte Bedeutung hat, überführt, diesen in der zweiten Stufe katalytisch mit Wasserstoff zu dem entsprechenden Amin der allgemeinen Formel 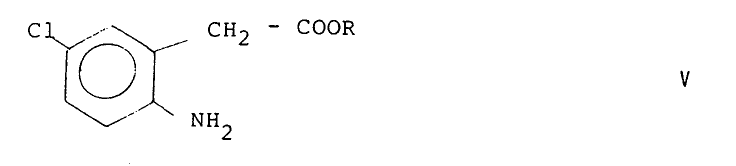 worin R die genannte Bedeutung hat, hydriert und dieses dann in der dritten Stufe in Gegenwart einer Säure zum Endprodukt gemäss Formel I cyclisiert.

2. Verfahren nach Patentanspruch 1, dadurch gekennzeichnet, dass man in der ersten Stufe als Base ein Alkaliamid in flüssigem Ammoniak verwendet.

3. Verfahren nach Patentanspruch 2, dadurch gekennzeichnet, dass man in der ersten Stufe das Alkaliamid in Gegenwart eines Alkohols der allgemeinen Formel
R - OH VI
worin R die genannte Bedeutung hat, einsetzt.

4. Verfahren nach mindestens einem der Patentansprüche 2 und 3, dadurch gekennzeichnet, dass man in der ersten Stufe als Alkohol tertiär-Butanol und als Alkaliamid Natriumamid verwendet.

5. Verfahren nach mindestens einem der Patentansprüche 1 bis 4, dadurch gekennzeichnet, dass man die Umsetzung in der ersten Stufe bei einer Temperatur von -30 bis -40°C durchführt.

6. Verfahren nach mindestens einem der Patentansprüche 1 bis 5, dadurch gekennzeichnet, dass man als Hydrierkatalysator in der zweiten Stufe Platin auf Kohle verwendet.

7. Verfahren nach mindestens einem der Patentansprüche 1 bis 6, dadurch gekennzeichnet, dass man die Hydrierung in der zweiten Stufe bei einem Druck von 5 bis 10 bar und bei einer Temperatur von 0 bis 55°C durchführt.

8. Verfahren nach mindestens einem der Patentansprüche 1 bis 7, dadurch gekennzeichnet, dass man als Säure in der dritten Stufe Toluol-4-sulfonsäure, Methansulfonsäure oder deren Hydrate verwendet.

9. Verfahren nach mindestens einem der Patentansprüche 1 bis 8, dadurch gekennzeichnet, dass man die Cyclisierung in der dritten Stufe bei einer Temperatur von 50°C bis Rückflusstemperatur durchführt.

10. Verfahren nach mindestens einem der Patentansprüche 1 bis 9, dadurch gekennzeichnet, dass man die Umsetzung ohne Isolation des Amins gemäss Formel V durchführt.

11. Verfahren zur Herstellung von Chlornitrobenzolessigsäurealkylestern der allgemeinen Formel 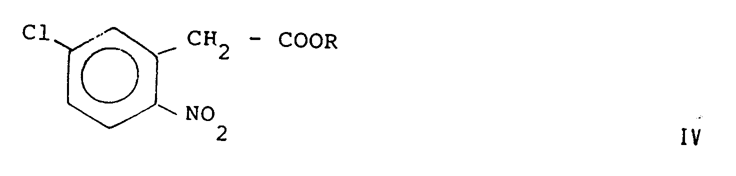 worin R die genannte Bedeutung hat, dadurch gekennzeichnet, dass man p-Chlornitrobenzol der Formel 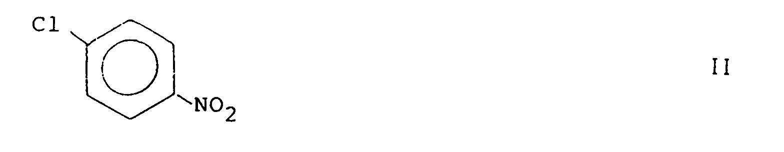 mit einem Chloressigsäurealkylester der allgemeinen Formel 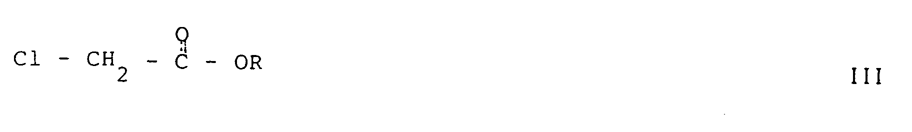 worin R die genannte Bedeutung hat, in Gegenwart eines Alkaliamids in flüssigem Ammoniak umsetzt.

12. Verfahren nach Patentanspruch 11, dadurch gekennzeichnet, dass man die Umsetzung in Gegenwart eines Alkohols der allgemeinen Formel
R - OH VI
worin R die genannte Bedeutung hat, durchführt.

13. Verfahren nach Patentanspruch 12, dadurch gekennzeichnet, dass man als Alkohol tertiär-Butanol und als Alkaliamid Natriumamid verwendet.

14. Verfahren nach mindestens einem der Patentansprüche 11 bis 13, dadurch gekennzeichnet, dass man die Umsetzung bei einer Temperatur von -30 bis -40°C durchführt.

## Claims

1. A process for preparing 5-chlorooxindole of the formula 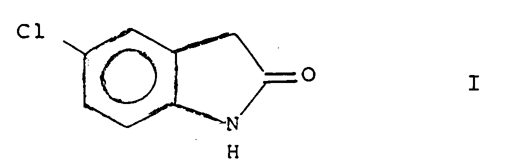 characterised in that, in a first step, p-chloronitrobenzene of the formula 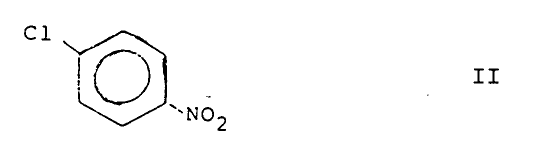 is reacted with an alkyl chloroacetate of the general formula
Cl - CH₂ - CO - OR III
in which R represents a C₁-C₇-alkyl group, branched or unbranched, in the presence of a base to give an alkyl chloronitrobenzenacetate of the general formula 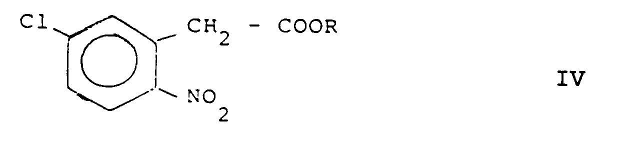 where R has the meaning given above, this is catalytically hydrogenated with hydrogen in the second step to give the corresponding amine of the general formula 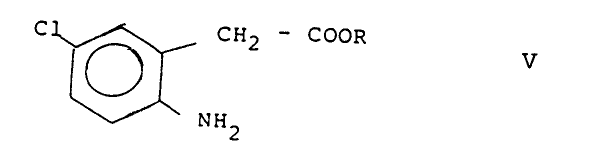 where R has the meaning given above, and this is then cyclised in the presence of an acid in the third step to give the end product in accordance with formula I.

2. A process according to Claim 1, characterised in that, in the first step, an alkali metal amide in liquid ammonia is used as the base.

3. A process according to Claim 2, characterised in that, in the first step, the alkali metal amide is used in the presence of an alcohol of the general formula
R-OH VI
in which R has the meaning given above.

4. A process according to at least one of Claims 2 and 3, characterised in that, in the first step, tertiary butanol is used as the alcohol and sodium amide is used as the alkali metal amide.

5. A process according to at least one of Claims 1 to 4, characterised in that the reaction in the first step is performed at a temperature of -30 to -40°C.

6. A process according to at least one of Claims 1 to 5, characterised in that platinum on carbon is used as hydrogenation catalyst in the second step.

7. A process according to at least one of Claims 1 to 6, characterised in that hydrogenation in the second step is performed at a pressure of 5 to 10 bar and at a temperature of 0 to 55°C.

8. A process according to at least one of Claims 1 to 7, characterised in that toluene-4-sulphonic acid, methanesulphonic acid or their hydrates are used as acids in the third step.

9. A process according to at least one of Claims 1 to 8, characterised in that cyclisation is performed in the third step at a temperature of 50°C to the reflux temperature of the mixture.

10. A process according to at least one of Claims 1 to 9, characterised in that the reaction is performed without isolating the amine in accordance with formula V.

11. A process for preparing alkyl chloronitrobenzenacetates of the general formula 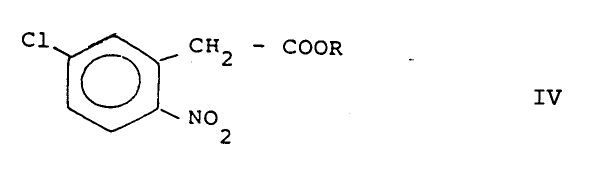 in which R has the meaning given above, characterised in that p-chloronitrobenzene of the formula 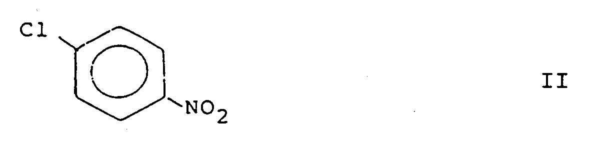 is reacted with an alkyl chloroacetate of the general formula
Cl - CH₂ - CO - OR III
in which R has the meaning given above, in the presence of an alkali metal amide in liquid ammonia.

12. A process according to Claim 11, characterised in that the reaction is performed in the presence of an alcohol of the general formula
R - OH VI
in which R has the meaning given above.

13. A process according to Claim 12, characterised in that tertiary butanol is used as the alcohol and sodium amide is used as the alkali metal amide.

14. A process according to at least one of Claims 11 to 13, characterised in that the reaction is performed at a temperature of -30 to -40°C.

## Revendications

1. Procédé pour la préparation du 5-chloroxindol de la formule 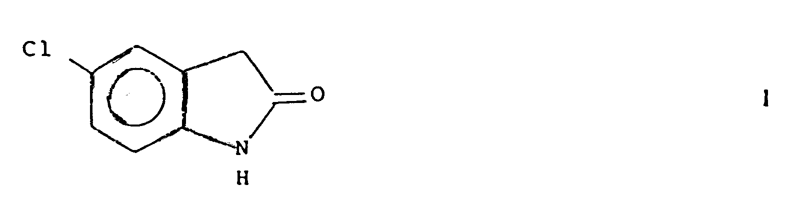 caractérisé en ce que l'on transforme dans la première étape le p-chloronitrobenzène de la formule 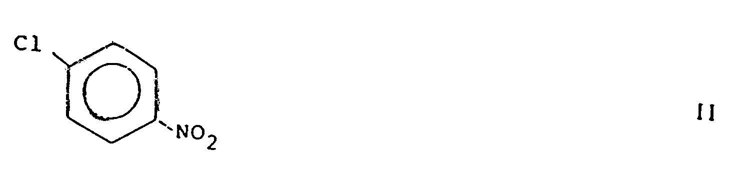 avec un alkylester de l'acide chloracétique de la formule générale 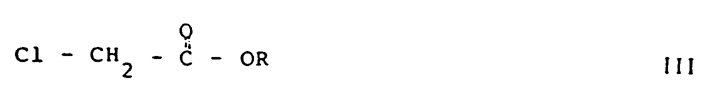 dans laquelle R signifie un groupe alkyle C₁-C₇ ramifié ou non ramifié, en présence d'une base en un alkylester de l'acide acétique de chloronitrobenzène de la formule générale 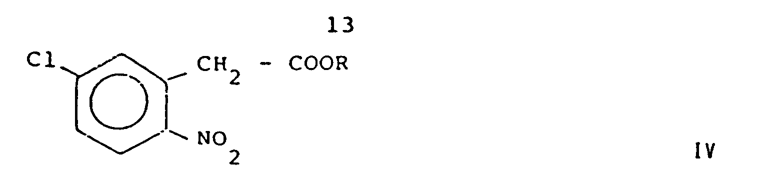 dans laquelle R a la signification indiquée, en ce que dans la deuxième étape, on transforme celui-ci par voie catalytique avec de l'hydrogène en l'amine correspondante de la formule générale 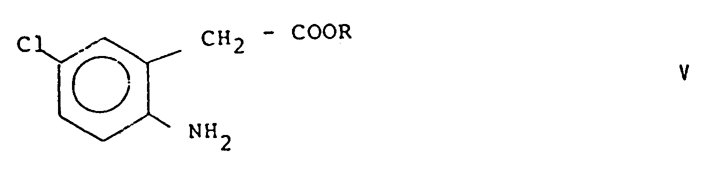 dans laquelle R a la signification indiquée, en ce que l'on hydrogène et cyclise celui-ci ensuite dans la troisième étape en présence d'un acide en le produit final selon la formule I.

2. Procédé selon la revendication 1, caractérisé en ce que l'on utilise comme base dans la première étape un amide alcalin dans de l'ammoniac liquide.

3. Procédé selon la revendication 2, caractérisé en ce que dans la première étape, on met en oeuvre un amide alcalin en présence d'un alcool de la formule générale
R - OH VI
dans laquelle R a la signification indiquée.

4. Procédé selon au moins l'une des revendications 2 et 3, caractérisé en ce que l'on utilise dans la première étape du butanol tertiaire en tant qu'alcool et un amide de sodium en tant qu'amide alcalin.

5. Procédé selon au moins l'une des revendications 1 à 4, caractérisé en ce que dans la première étape, on conduit la réaction à une température de -30 à -40°C.

6. Procédé selon au moins l'une des revendications 1 à 5, caractérisé en ce qu'en tant que catalyseur d'hydrogénation, on utilise dans la deuxième étape du platine sur du carbone.

7. Procédé selon au moins l'une des revendications 1 à 6, caractérisé en ce que dans la deuxième étape, on conduit l'hydrogénation à une pression de 5 à 10 bars et à une température de 0 à 55°C.

8. Procédé selon au moins l'une des revendications 1 à 7, caractérisé en ce que dans la troisième étape, on utilise en tant qu'acide, l'acide de toluol-4-sulfonique, l'acide méthane sulfonique ou leurs hydrates.

9. Procédé selon au moins l'une des revendications 1 à 8, caractérisé en ce que l'on conduit la cyclisation dans la troisième étape à une température de 50°C jusqu'à la température de reflux.

10. Procédé selon au moins l'une des revendications 1 à 9, caractérisé en ce que l'on conduit la réaction sans isoler l'amine selon la formule V.

11. Procédé pour la préparation d'alkylesters de l'acide acétique de chloronitrobenzène de la formule générale 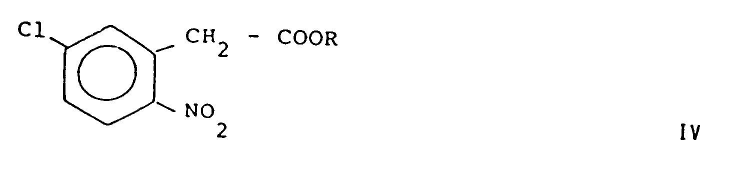 dans laquelle R a la signification indiquée, caractérisé en ce que l'on met en réaction le p-chloronitrobenzène de la formule 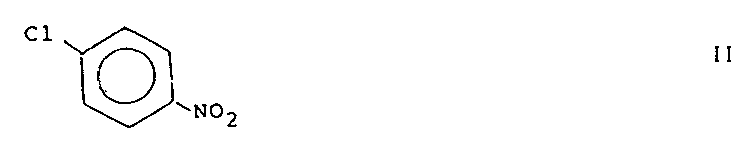 avec un alkylester de l'acide chloracétique de la formule générale 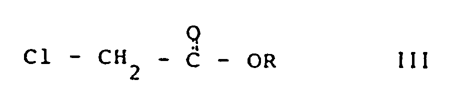 dans laquelle R à la signification indiquée, en présence d'un amide alcalin dans l'ammoniac liquide.

12. Procède selon la revendication 11, caractérisé en ce que l'on conduit la réaction en présence d'un alcool de la formule générale
R - OH VI
dans laquelle R a la signification indiquée.

13. Procédé selon la revendication 12, caractérisé en ce qu'en tant qu'alcool, on utilise du butanol tertiaire et en tant qu'amide alcalin, de l'amide de sodium.

14. Procédé selon au moins l'une des revendications 11 à 13, caractérisé en ce que l'on conduit la réaction à une température de -30 à -40°C.
